# FASCICULE DE BREVET EUROPEEN

(11) **EP 1 245 583 B1**
(45) Date de publication et mention de la délivrance du brevet: **19.09.2007**
(21) Numéro de dépôt: 02290784.4
(22) Date de dépôt: 28.03.2002
(51) Int. Cl.: C08B 30/18, A23L 1/09, A23L 1/308, A23L 2/52, A23L 1/29, A21D 2/18, A23G 3/00, A23L 1/164, A23C 9/154

(54) **Denrée alimentaire à libération d'énergie prolongée**
Lebensmittel mit verlängerter Energiefreisetzung
Food product with sustained energy release

(30) Priorité: 30.03.2001 FR 0104412
(43) Date de publication de la demande: 02.10.2002
(73) Titulaire: Roquette Frères, 62136 Lestrem (FR)
(72) Inventeur: Saniez, Marie-Hélène, 59350 Saint-Andre (FR)
(74) Mandataire: Boulinguiez, Didier

(56) Documents cités:
- EP-A- 1 006 128
- WO-A-95/02969
- US-A- 6 114 379
- US-A- 6 159 943

## Description

La présente invention a pour objet une composition nutritive destinée notamment aux sportifs, susceptible de constituer un apport énergétique prolongé. Plus précisément, l'invention a pour objet une composition nutritive à libération d'énergie prolongée, contenant un saccharide particulier. L'invention a également pour objet une denrée alimentaire contenant ladite composition nutritive.

L'aptitude aux exercices de longue durée est limitée par la teneur en glycogène du muscle. Il existe en effet une relation linéaire entre cette teneur au niveau des membres inférieurs et le délai d'épuisement lors d'exercices durant de quelques minutes à quelques heures. C'est pourquoi parmi les objectifs de l'entraînement et de la préparation nutritionnelle des sportifs figurent l'augmentation avant l'épreuve des réserves de glycogène dans les muscles sollicités par l'exercice et l'économie du glycogène musculaire pendant l'épreuve. Il a été démontré que l'alimentation glucidique avant, pendant et après l'exercice était déterminante dans la gestion des réserves de glycogène musculaire. Les stratégies nutritionnelles dans la préparation, pendant le déroulement et lors de la récupération d'épreuves intenses et de longue durée tient compte des cinétiques et facteurs de synthèse du glycogène musculaire. Ces stratégies sont nombreuses et continuent d'évoluer. Elles comportent en général trois volets : la préparation quelques jours avant l'épreuve dont l'objectif est la charge optimale des glycogènes musculaire et hépatique, la préparation dans l'heure précédant l'exercice dont l'objectif est de poursuivre la mise en réserve du glycogène hépatique, et le contrôle de la glycogénolyse musculaire pendant l'épreuve. Les apports sont différents suivant le volet considéré. En ce qui concerne les apports immédiatement avant l'effort et pendant l'effort, auxquels la présente invention se rapporte, il est généralement conseillé de consommer des carbohydrates qui sollicitent au minimum la sécrétion d'insuline pour ne pas perturber les modifications hormonales nécessaires aux adaptations métaboliques énergétiques en début d'exercice. En cas d'apport excessif de glucose, par exemple, le sujet risque une hyper puis une hypoglycémie réactionnelle dans la première demi-heure d'exercice par hyper insulinémie, qui se traduit par une fringale, une fatigue pouvant aller jusqu'au malaise et une nécessité de ralentir l'allure, voire d'arrêter. Ce même phénomène a été observé avec des carbohydrates de type maltodextrines. Une solution de fructose est actuellement considérée comme la boisson optimale juste avant l'effort, en solution jusqu'à 35g/l. Le fructose est moins insulino-sécréteur et son absorption intestinale est plus lente.

Le document US 6 114 379 divulgue des barres énergétiques, qui contiennent 5% de maltodextrines (10 D.E.) et 12% de fructose cristallin, ou 8% de maltodextrine (10 D.E.) et 13% de fructose cristallin, ou 6% de maltodextrine (10 D.E.) et 15% de fructose cristallin (cf. exemple 2).

Le document US 6 159 943 divulgue des formules à libération d'énergie prolongée qui contiennent de la maltodextrine et du fructose et qui peuvent contenir aussi du ribose.

Partant de ce constat de l'état de la technique et cherchant à mettre au point une composition énergétique destinée à un apport juste avant et pendant l'effort, la Demanderesse a trouvé, après de nombreuses recherches, qu'il était possible d'atteindre cet objectif des lors que la composition énergétique comprenait un saccharide particulier, capable contre toute attente de constituer un apport d'énergie prolongé, c'est à dire dès sa consommation et tout au long de l'effort, sans présenter l'inconvénient de générer une augmentation rapide de la glycémie suivie d'une hypoglycémie brutale.

L'invention a donc pour objet l'utilisation d'une composition nutritive à libération d'énergie prolongée caractérisée en ce qu'elle comprend des maltodextrines branchées présentant entre 15 et 35% de liaisons glucosidiques 1-6, une teneur en sucres réducteurs inférieure à 20%, un indice de polymolécularité inférieur à 5 et une masse moléculaire moyenne en nombre Mn au plus égale à 4500 g/mole pour apporter de l'énergie à libération prolongée sans engendrer des périodes d'hypoglycémie lorsque la composition est ingérée par un individu.

Par maltodextrines branchées on entend au sens de la présente invention les maltodextrines décrites dans la demande de brevet EP 1.006.128 dont la Demanderesse est titulaire. Ces maltodextrines branchées présentent un caractère d'indigestibilité qui a pour conséquence de diminuer leur pouvoir calorique, en empêchant leur assimilation au niveau de l'intestin grêle. Leur faible teneur en molécules de faible degré de polymérisation (« DP ») contribue également à leur faible caloricité. Leur teneur en liaisons glucosidiques 1-6 comprise entre 22 et 35% a pour conséquence d'abaisser leur pouvoir cariogène en réduisant leur assimilation par les micro-organismes de la cavité buccale. Ce taux élevé en liaisons 1-6 leur confère également des propriétés prébiotiques tout à fait particulières : il est en effet apparu que les bactéries du cæcum et du colon de l'homme et des animaux, telles que les bactéries butyrogênes, lactiques ou propioniques métabolisent des composés hautement branches. D'autre part, ces maltodextrines branchées favorisent le développement des bactéries bifidogènes au détriment des bactéries indésirables. Il en résulte des propriétés tout à fait bénéfiques pour la santé du consommateur. Ces maltodextrines branchées présentent en outre une teneur en liaisons glucosidiques 1-4 résiduelles relativement élevée. Cette teneur peut être comprise entre 42 et 50%, teneur qui, à la connaissance de la Demanderesse, n'a encore jamais été décrite en combinaison avec une teneur en liaisons 1-6 comprise entre 22 et 35%. Les maltodextrines branchées présentent ainsi un ratio de liaisons 1-6 / 1-4 d'environ 0,6.

L'indice de polymolécularité est donné par le rapport des masses moléculaires en poids sur les masses moléculaires en nombre, ces dernières étant mesurées par exemple par chromatographie de perméation de gel.

Lesdites maltodextrines branchées présentent un indice de polymolécularité inférieur à 5, pour une masse moléculaire en nombre (Mn) de 500 à 4500 g/mole.

Leur masse moléculaire en poids (MW) est ainsi comprise entre 2000 et 6000 g/mole environ.

Des dextrines de haut poids moléculaire (720000 g/mole) et présentant un taux de liaisons 1-6 / 1-4 de 0,02 à 0,08 ont été proposées dans le document WO 95/22562 pour la préparation des sportifs avant et après l'effort. Ces dextrines sont en fait destinées à renforcer le stockage de glycogène quelques jours avant l'effort de la même manière que des aliments contenant des glucides complexes (pâtes, riz).

Les maltodextrines branchées selon l'invention étant faiblement digérées dans l'intestin grêle, elles apportent lentement de l'énergie sous forme de glucose à l'individu qui les consomme. Ce glucose, unité constitutive des maltodextrines branchées, apparaît alors lentement dans la circulation sanguine, et n'entraîne pas d'hypoglycémie après ingestion. L'apport d'énergie se poursuit dans le gros intestin où les maltodextrines branchées sont fermentées en acides gras à courte chaîne (dits acides gras volatils) participant au métabolisme énergétique. Ce n'est pas le cas du glucose ou d'autres glucides simples qui eux, sont complètement absorbés dans l'intestin grêle.

Selon une variante préférée de l'invention, lesdites maltodextrines branchées présentent une teneur en sucres réducteurs comprise entre 2 et 5% et un Mn compris entre 2000 et 3000 g/mole.

Selon une autre variante préférée de l'invention, tout ou partie desdites maltodextrines branchées sont hydrogénées.

La composition nutritive selon l'invention est en premier lieu destinée aux sportifs pratiquant des exercices d'endurance, mais elle est également tout à fait adaptée à l'apport énergétique prolongé de tout individu y compris les enfants, ayant besoin de cette source énergétique en différentes occasions.

La composition nutritive selon l'invention peut être ingérée de différentes manières. Elle peut en effet se présenter sous forme de poudre, conditionnée en sachets et éventuellement adjointe d'additifs tels que par exemple sels minéraux, vitamines, arômes, colorants, en vue d'être ingérée telle quelle ou dissoute extemporanément dans une boisson. Elle peut également se présenter sous forme de sirop prêt à l'emploi

La teneur en maltodextrines branchées de la dite composition nutritive est comprise entre 50 et en poids, de préférence entre 60 et 99,9% et plus préférentiellement encore entre 70 et 99,9% en poids.

La composition nutritive selon l'invention peut avantageusement comprendre un mélange desdites maltodextrines branchées avec tous types d'oses hypoglycémiques et/ou hypoinsulinémiques, comme par exemple le fructose ou le tagatose. Selon une variante avantageuse, ladite composition nutritive comprend un mélange desdites maltodextrines branchées avec un ou plusieurs pentoses, ce mélange pouvant être formulé sous forme de poudres ou sous forme liquide, sachant que dans ce dernier cas, la stabilité bactériologique des maltodextrines branchées sous forme liquide s'en trouve avantageusement nettement améliorée. On peut par exemple réaliser des mélanges de maltodextrines branchées selon l'invention avec par exemple des sirops de fructose comme le MELIOSE^{®} 700 commercialisé par la Demanderesse. Les proportions du mélange varient en fonction de la formulation finale à laquelle on destine ledit mélange. A titre d'exemple on peut réaliser des mélanges comprenant jusqu'à 50 % de fructose, sous forme de sirops d'environ 70% de matière sèche.

La composition nutritive selon l'invention peut également être incorporée à une denrée alimentaire consistant notamment en une barre énergétique, une barre de céréales, un biscuit, une boisson, un complément diététique, une composition céréalière pour petit déjeuner, un comprimé, une confiserie, un yaourt ou une crème dessert. Préférentiellement, ladite denrée alimentaire est une barre, un biscuit, une boisson, une confiserie. La denrée alimentaire selon l'invention comprend avantageusement 0,5 à 75% et de préférence 0,5 à 30% de ladite composition nutritive.

La composition nutritive selon l'invention fournit de l'énergie se traduisant par une élévation de la glycémie post-prandiale sans engendrer de période d'hypoglycémie, cette énergie étant libérée dans l'organisme de manière prolongée, c'est à dire au moment de sa consommation jusque longtemps après.

L'invention sera mieux comprise à la lecture des exemples qui suivent et de la figure qui s'y rapporte, donnés à titre illustratif et non limitatif.

### EXEMPLE 1 : ETUDE DE LA GLYCEMIE ET DE L'INSULINEMIE.

6 individus sains ont reçu soit 50g de glucose soit 50g d'une composition nutritive contenant 95% en poids sec de maltodextrines branchées dont la composition est la suivante :

| | |
|---|---|
| Sucres réducteurs | 2,3 |
| Mn (g/mole) | 2480 |
| Mw (g/mole) | 5160 |
| Liaison 1,2 (%) | 10 |
| Liaison 1,3 (%) | 12 |
| Liaison 1,4 (%) | 49 |
| Liaison 1,6 (%) | 29 |

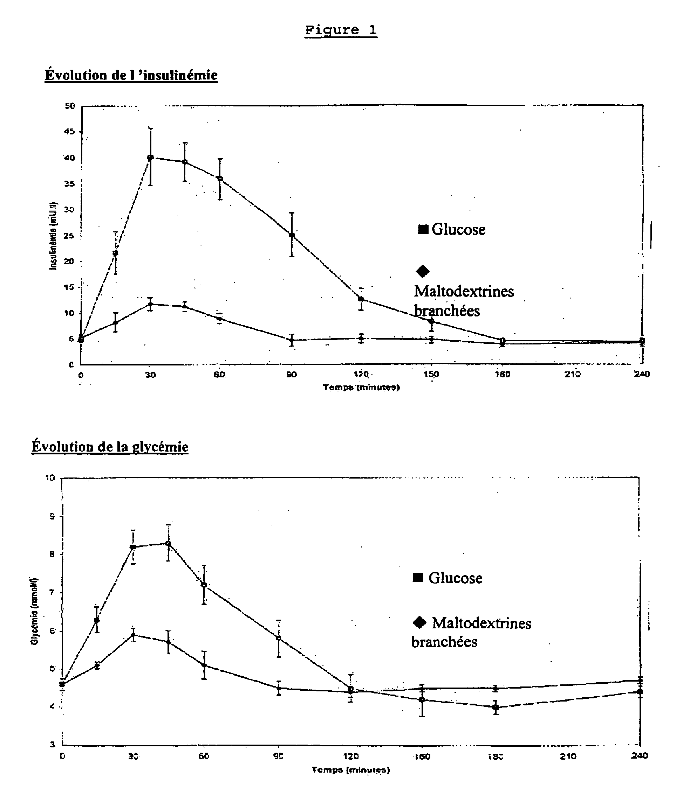

La glycémie et l'insulinémie sont mesurées avant consommation du glucide, puis aux temps 15,30,45,60,90,120,150,180,et 240 minutes après ingestion. Les courbes de glycémie et d'insulinémie en fonction du temps sont établies pour chaque glucide et sont représentées en figure 1.
➢ La glycémie et l'insulinémie maximales sont atteintes 30 minutes après l'ingestion du glucide, quel qu'il soit.
➢ La glycémie et l'insulinémie reviennent à la ligne de base en 90 minutes chez les individus ayant reçu la composition nutritive selon l'invention, contre 120 minutes pour les individus ayant reçu le glucose.
➢ La glycémie reste constante du temps 90 au temps 240 chez les individus ayant reçu la composition nutritive selon l'invention, alors qu'elle diminue encore dans le cas du glucose pour ne remonter qu'au temps 240 sans avoir atteint la ligne de base. L'insulinémie diminue encore dans le cas du glucose, alors qu'elle reste constante, à la ligne de base dans le cas des maltodextrines branchées.

Ces résultats démontrent que l'ingestion d'une composition nutritive conforme à l'invention entraîne une élévation de la glycémie post-prandiale pouvant donc apporter de l'énergie sans engendrer d'hypoglycémie. L'insulinémie est quant à elle nettement inférieure par rapport au glucose.

### EXEMPLE 2 : PREPARATION DE DENREES ALIMENTAIRES COMPRENANT LA COMPOSITION NUTRITIVE SELON L'EXEMPLE 1.

### 1) Barre de céréales.

### Recette du caramel liant

| | Ingrédient mis en oeuvre (en poids) | Composition du produit fini (%) |
|---|---|---|
| Lait en poudre | 101 | 10 |
| Saccharose | 300 | 32 |
| Composition nutritive | 126 | 12.7 |
| NaCl | 4 | 0.4 |
| Eau | 40 | |
| NEOSORB 70/70* | 118 | 8.8 |
| FLOLYS E7081S* | 132 | 11 |
| M.G.V.** COSE | 174 | 18 |
| Lécithine de soja | 5 | 0.5 |
| Humidité résiduelle | | 6.6 |
| Total | 1000 | 100 |

| | | |
|---|---|---|
| * Commercialisé par la Demanderesse ** Matière grasse végétale | | |

### Mode opératoire

➢ Mélanger le lait entier en poudre avec le saccharose (M1)
➢ Mélanger l'eau, le NEOSORB 70/70 et le FLOLYS E7081S (M2)
➢ Mélanger la matière grasse végétale (préalablement fondue à 50°C), la lécithine et les maltodextrines branchées (M3)
➢ Sous agitation ajouter le mélange M1 au mélange M2 puis cuire à feu doux jusqu'à 110°C
➢ Lorsque la température de 110°C est atteinte, verser progressivement le mélange M3
➢ Une chute de température (5°C environ) et une augmentation de la viscosité du mélange final sont alors observées.
➢ Toujours à feu doux et en agitant fortement cuire le mélange final jusqu'à 110°C.
➢ Mélanger le caramel aux céréales en respectant les proportions prescrites (75/25).
➢ Couler sur marbre
➢ Refroidir, découper, envelopper.

### 2) Boisson.

On prépare une boisson gazeuse contenant la composition nutritive conforme à l'invention comprenant 95% en poids de maltodextrines branchées.

Quantités en grammes pour 1 litre de boisson

| | **Boisson selon l'invention** |
|---|---|
| **Composition nutritive** | 73,68 |
| **Aspartame** | 0,118 |
| **Acésulfame K** | 0,118 |
| **Arôme citron** | 0,7 |
| **Acide citrique (sec)** | 1,9 |
| **Benzoate de sodium (sec)** | 0,09 |
| **Eau à 4°C** | qsp 11 |

Aspartame : NUTRASWEET
Acesulfame K : HOESCHT
Arôme citron : AG 31711 (QUEST)

On prépare 0,5 litres d'eau gazéifiée. On ajoute ensuite les édulcorants et la composition nutritive selon l'invention. On incorpore ensuite le reste des ingrédients et on ajoute de l'eau jusqu'à un volume de 1 litre.

### 3) Biscuit.

On prépare des biscuits contenant la composition nutritive conforme à l'invention, comprenant 95% en poids sec de maltodextrines branchées de composition similaire à celle de l'exemple 1.

**Quantités pour 100 g de pâte :**

| Ingrédient | Biscuit selon l'invention |
|---|---|
| Farine | 55,8 |
| Matière grasse | 12,4 |
| Saccharose | 15,1 |
| Composition nutritive | 6,2 |
| Eau | 9,3 |
| Bicarbonate d'ammonium | 0,3 |
| Bicarbonate de soude | 0,2 |
| Pyrophosphate de soude | 0,2 |
| Sel | 0,3 |
| Vanille | 0,1 |
| Lécithine | 0,1 |

### Mode opératoire :

- Peser l'eau, le bicarbonate d'ammonium et le bicarbonate de soude. Mélanger pendant 5 minutes dans un pétrin Hobart à vitesse 1.
- Ajouter la matière grasse et la lécithine de soja, et agiter une minute à vitesse 1, puis 4 minutes à vitesse 2.
- Peser le reste des poudres (farine, saccharose, composition nutritive, pyrophosphate acide de soude, sel et arôme vanille). Les mélanger, puis les ajouter dans le pétrin. Agiter 10 minutes à vitesse 1, avec une interruption pour racler les bords du pétrin et la pale d'agitation.
- Former les biscuits à la mouleuse rotative, et les disposer sur une plaque de cuisson.
- Porter au four rotatif à 200°C pendant 10 minutes.
- Laisser refroidir à 25°C.

### 4) Crème dessert.

**Ingrédients mis en oeuvre:**

| **ingrédients** | **Pourcentage en poids mis en oeuvre** |
|---|---|
| Lait écrémé en poudre | 9 |
| Composition nutritive | 5,7 |
| Acesulfame K | 0,04 |
| Amidon * | 3 |
| Carraghénane | 0,02 |
| Arôme vanille | 0,01 |
| Eau | 82,23 |
| total | 100 |

| | |
|---|---|
| Amidon : CLEARAM^{®}CH3010 commercialisé par la Demanderesse. | |

25 litres d'eau sont versés dans la cuve d'agitation d'un échangeur tubulaire (VOMATEC B.V.) puis la poudre de lait est ajoutée. On la laisse s'hydrater pendant 20 minutes.

La cuve est chauffée à 40°C et le reste des ingrédients est ajouté.

Après une période d'agitation d'environ 20 minutes, le mélange est stérilisé dans l'échangeur tubulaire, où il subit un traitement thermique de 130°C pendant 1 minute 30.

La crème dessert est récupérée à la sortie de l'appareil dans des pots stériles.

## Revendications

1. Utilisation d'une composition nutritive comprenant entre 50 et 99,9%, de préférence 60 à 99,9% et plus préférentiellement encore entre 70 et 99,9% en poids de maltodextrines branchées présentant entre 15 et 35% de liaisons glucosidiques 1-6, une teneur en sucres réducteurs inférieure à 20%, un indice de polymolécularité inférieur à 5 et une masse moléculaire moyenne en nombre Mn au plus égale à 4500 g/mole, pour apporter de l'énergie à libération prolongée sans engendrer de période d'hypoglycémie lorsque la composition est ingérée par un individu.

2. Utilisation selon la revendication 1, **caractérisée en ce que** lesdites maltodextrines branchées présentent une teneur en sucres réducteurs comprise entre 2 et 5% et un Mn compris entre 2000 et 3000 g/mole.

3. Utilisation selon l'une ou l'autre des revendications 1 et 2, **caractérisée en ce que** la composition nutritive comprend un mélange desdites maltodextrines branchées avec au moins un ose hypoinsulinémique.

## Claims

1. Use of a nutritive composition comprising between 50 to 99.9%, preferably between 60 and 99.9% and most preferably between 70 and 99.9% by weight of branched maltodextrins having between 15 and 35% of 1-6 glucoside linkages, a reducing sugar content of less than 20%, a polymolecularity index of less than 5 and a number-average molecular mass Mn at most equal to 4500 g/mol for supplying prolonged energy release without causing a period of hypoglycemia when it is ingested by an individual.

2. Use according to claim 1, **characterized in that** said branched maltodextrins have a reducing sugar content of between 2 and 5% and a Mn of between 2000 and 3000 g/mol.

3. Use according to claim 1 or 2, **characterized in that** the nutritive composition comprises a mixture of said branched maltodextrins with at least one hypoinsulinaemic sugar.

## Patentansprüche

1. Verwendung einer Nährstoff-Zusammensetzung, die zwischen 50 und 99,9 Gew.-%, vorzugsweise zwischen 60 und 99,9 Gew.-%, noch bevorzugter zwischen 70 und 99,9 Gew.-% an verzweigten Maltodextrinen enthält, die zwischen 15und 35 % glukosidischer Bindungen 1-6, einen Gehalt von reduzierten Zuckern von weniger als 20 %, einen Polymolekularitätsindex von weniger als 5 und eine mittlere Molmassezahl Mn von höchstens gleich 4.500 g / Mol aufweisen, zum Zuführen von Energie mit verlängerter Freisetzung ohne Unterzuckerungsperiode, wenn die Zusammensetzung von einem Individuum eingenommen wird.

2. Verwendung nach Anspruch 1, **dadurch gekennzeichnet, dass** die besagten verzweigten Maltodextrine einen Gehalt an reduzierten Zuckern aufweisen, der zwischen 2 und 5 % liegt, und einen Mn-Wert zwischen 2000 und 3000 g / Mol.

3. Verwendung nach dem einen oder dem anderen der Ansprüche 1 und 2, **dadurch gekennzeichnet, dass** die Nährstoff-Zusammensetzung eine Mischung der besagten verzweigten Maltodextrine mit mindestens einem Hypoinsulin-Monosaccharid enthält.
